# EUROPEAN PATENT SPECIFICATION

(11) **EP 3 943 950 B1**
(45) Date of publication and mention of the grant of the patent: **14.02.2024**
(21) Application number: 20783285.8
(22) Date of filing: 26.03.2020
(51) Int. Cl.: G01N 35/10, G01N 1/14

(54) **LIQUID EXTRACTION DEVICE AND LIQUID EXTRACTION METHOD**
VORRICHTUNG UND VERFAHREN ZUR EXTRAKTION VON FLÜSSIGKEITEN
DISPOSITIF ET PROCÉDÉ D'EXTRACTION DE LIQUIDE

(30) Priority: 29.03.2019 CN 201910251282
(43) Date of publication of application: 26.01.2022
(73) Proprietor: Hemoassay Science and Technology (Suzhou) Co., Ltd., Suzhou, Jiangsu 215000 (CN)
(72) Inventor: XIAO, Jian, Suzhou, Jiangsu 215000 (CN); HU, Xi, Suzhou, Jiangsu 215000 (CN); CHEN, Jienian, Suzhou, Jiangsu 215000 (CN); WANG, Tao, Suzhou, Jiangsu 215000 (CN); JIANG, Feng, Suzhou, Jiangsu 215000 (CN)
(74) Representative: Wang, Bo
(86) International application number: PCT/CN2020/081460
(87) International publication number: WO 2020/200047

(56) References cited:
- WO-A1-2018/007290
- US-A- 4 000 973

## Description

### TECHNICAL FIELD OF THE INVENTION

The invention relates to a thromboelastometer, in particular to a liquid-taking device and a liquid-taking method of the thromboelastometer.

### DESCRIPTION OF THE PRIOR ART

A thromboelastometer is a medical device used to test whether blood can coagulate normally. It is generally necessary to detect the patient's blood coagulation data through the thrombo elastometer before performing an operation, and judge whether the patient's blood coagulation process is normal based on the detected blood coagulation data. Only when the blood is able to coagulate normally, the patient can be operated on. If the blood coagulation process is abnormal, the patient's blood will not be able to coagulate normally during the operation, leading to difficulty in hemostasis, which may endanger the patient's life.

The physical properties of testing a blood clot are based on the following principles. A special cylindrical cup statically containing blood rotates at a low angular speed of 4.75, and a testing rod immersed in the blood sample is used to detect the movement of the blood sample. After the cup adheres to the testing rod through a fibrin platelet complex, the rotational force generated by rotating the cup can be transmitted to the testing rod in the blood sample. The strength of the fibrin platelet complex can influence the movement amplitude of the testing rod, so that a strong blood clot can synchronize the movements of both the testing rod and the cup. Therefore, the movement amplitude of the testing rod is directly related to the strength of the formed blood clot. In the case that the blood clot retracts or dissolves, the joint between the testing rod and the blood clot is released, so the movement of the cup is no longer transmitted to the testing rod, the rotation of which is detected by the sensor.

As for the current thromboelastometer, when detecting blood coagulation data, it is necessary to manually add blood and reagents to a testing cup, which requires a lot of time for operators, resulting in high labor intensity and low test efficiency for detecting blood coagulation data. In addition, manual addition depends greatly on the technical level of operators, which affects test accuracy.

WO 2018/007290 A1 discloses a known pipetting device.

### SUMMARY OF THE INVENTION

A objective of the invention is to overcome the defects of the prior art and provide a liquid-taking device and a liquid-taking method that achieve automatically suctioning blood and reagents.

To achieve the above object, the present invention proposes a liquid-taking device according to claim 1.

Preferably, both the blood suction-driving mechanism and the reagent suction-driving mechanism are fixed on a base, the base is rotationally connected with rotationally-driving mechanism.

Preferably, the rotationally-driving mechanism includes a third driving source and a rotating shaft connected with the third driving source, the rotating shaft is fixedly connected to both the blood suction-driving mechanism and the reagent suction-driving mechanism.

Preferably, both the first gear assembly and the second gear assembly include a first gear and a second gear that mesh perpendicularly with each other, the first gear is connected with the driving source, and the second gear is connected with the screw rod.

Preferably, both the blood suction-driving mechanism and the reagent suction-driving mechanism further include a first sliding rail, both the first sliding stand and the second sliding stand move along respective corresponding sliding rail, respectively, driven by respective corresponding driving source.

Preferably, the first sliding stand is connected with one blood-suctioning member, and the second sliding stand is connected with a plurality of reagent-suctioning members.

This present invention also proposes a liquid-taking method according to claim 10.

Preferably, S1 specifically includes, the rotationally-driving mechanism driving the blood suction-driving mechanism to move to the blood sample testing tube, the blood suction-driving mechanism driving the blood-suctioning member to extend into the corresponding blood sample testing tube to suction a blood sample, after finishing suctioning, driving the blood-suctioning member to extend out of the blood sample testing tube, and then the reagent suction-driving mechanism being driven by the rotationally-driving mechanism to move to the testing cup, and being prepared to add the blood sample into the testing cup.

Preferably, S2 specifically includes, the rotationally-driving mechanism driving the reagent suction-driving mechanism to move to the reagent testing tube, the reagent suction-driving mechanism driving the reagent-suctioning member to extend into the corresponding reagent testing tube to suction a reagent, after finishing suctioning, driving the reagent-suctioning member to extend out of the reagent testing tube, and then the reagent suction-driving mechanism being driven by the rotationally-driving mechanism to move to the testing cup, and being prepared to add the reagent into the testing cup.

The beneficial effects of the present invention are as follows.
1. Setting up a plurality of driving mechanisms realizes automatically driving the addition of a blood sample and a reagent during detecting the blood coagulation data, and improving test efficiency and test accuracy.
2. Setting up a sliding stand shortens the height of the entire suction-driving mechanism for blood and reagents, thereby reducing the height and volume of the liquid-taking device, and achieving miniaturization.

### BRIEF DESCRIPTION OF THE DRAWINGS

FIG.1 is a schematic diagram of 3D structure of the device according to the invention.
FIG.2 is a schematic diagram of the partial enlarged structure in FIG.1.
FIG.3 is a front view of the structure in FIG. 1.
FIG.4 is a flow chart of the method according to the invention.

Wherein:
1- blood suction-driving mechanism; 11- first driving source; 12- first screw rod; 13- first sliding stand; 131- first fixing bump; 14- first gear; 15- second gear; 2- reagent suction-driving mechanism; 21- second driving source; 22- second screw rod; 23- second sliding stand; 231-second fixing bump; 3- rotationally-driving mechanism; 31- third driving source; 32- rotating shaft; 4- blood-suctioning member; 5- reagent-suctioning member; 6- first mounting plate; 61- first sliding rail; 62- second sliding rail; 63- liquid level sensor; 7- second mounting plate; 8- rotary bearing; 9- integrated rod; 91- connection opening; 10- base.

### DETAILED DESCRIPTION OF THE INVENTION

The technical solutions of the embodiments in the invention will be clearly and completely described below in combination with the figures in the invention.

A liquid-taking device disclosed in the invention realizes automatic driving during adding blood samples and reagents through the cooperation of three designed driving mechanisms (a blood suction-driving mechanism 1, a reagent suction-driving mechanism 2 and a rotationally-driving mechanism 3).

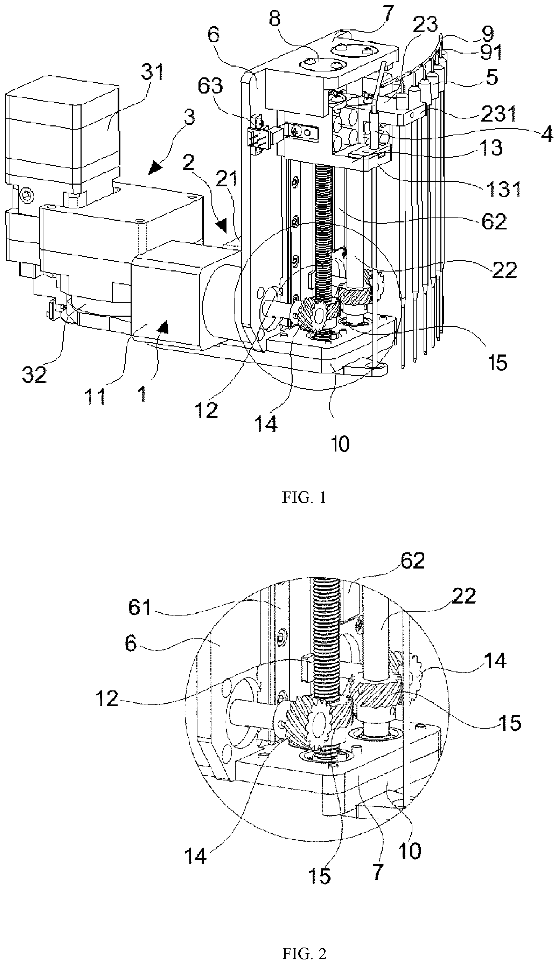

As shown in FIGs.1-3, the liquid-taking device disclosed in the embodiments of the invention includes a blood suction-driving mechanism 1, a reagent suction-driving mechanism 2, and a rotationally-driving mechanism 3, wherein the blood suction-driving mechanism 1 is connected to a blood-suctioning member 4, and the blood-suctioning member 4 is driven by it to extend into or out of a blood sample testing tube (not shown in the figures), the reagent suction-driving mechanism 2 is connected to a reagent-suctioning member 5, the reagent-suctioning member 5 is driven by it to extend into or out of a reagent testing tube (not shown in the figures), the rotationally-driving mechanism 3 is connected to both the blood suction-driving mechanism 1 and the reagent suction-driving mechanism 2, used to match with both the mechanisms 1, 2 to drive both to rotate in the first direction, and move to the corresponding blood sample testing tube and the reagent testing tube, respectively. The first direction in this embodiment is the horizontal direction.

Specifically, the blood suction-driving mechanism 1 includes a first driving source 11, a first gear assembly, a first screw rod 12 and a first sliding stand 13, wherein the first driving source 11 is fixed on a first mounting plate 6 vertically arranged, specifically fixed to the lower end of the first mounting plate 6, the first driving source 11 can be a first motor 11, when implemented, and the driving shaft of the first motor 11 horizontally passes through the first mounting plate 6.

The first gear assembly includes a first gear 14 and a second gear 15, wherein the first gear 14 is fixed to the top end of the driving shaft of the first motor 11 (that is, the end that passes through the first mounting plate 6 ), driven to rotate axially by the driving shaft of the first motor 11, the second gear 15 encircles the first screw rod 12 and meshes perpendicularly with the first gear 14, that is, the second gear 15 rotates in a radial direction perpendicular to the axial direction while the first gear 14 is rotating, that is, achieves a 90°turn.

The first screw rod 12 is vertically arranged, that is, parallel or approximately parallel to the plane where the first mounting plate 6 is located, in this embodiment, it is threadedly connected to the second gear 15 and can be driven with the rotation of the second gear 15 to rotate in a radial direction perpendicular to the rotation direction with respect to the second gear 15. Specifically, in this embodiment, the upper and lower ends of the first screw rod 12 are rotatably connected to a second mounting plate 7, respectively, and the second mounting plate 7 and the first mounting plate 6 can be integrally formed or arranged separately. When implemented, the rotational connection between both the ends of the first screw rod 12 and the second mounting plate 7 can be achieved through a rotary bearing 8.

The first sliding stand 13 is slidably connected to the first screw rod 12, and moves up and down along with the rotation of the first screw rod 12. Specifically, in this embodiment, the first sliding stand 13 is located between both the second mounting plates 7 and is arranged at the upper end of the first screw rod. Preferably, the first sliding stand 13 is also guided by a first sliding rail 61 to move in the vertical direction, and a first sliding groove matching with the first sliding rail 61 is arranged on the inner surface of the first sliding stand 13 abutting onto the first sliding rail 61 (not shown in the figures). In this embodiment, the first sliding rail 61 is vertically arranged on the first mounting plate 6.

The outer surface of the first sliding stand 13 (the side opposite to the first sliding groove) protrudes outwards to form a first fixing bump 131, and the blood-suctioning member 4 vertically passes through the first fixing bump 131 and is fixed inside the first fixing bump 131. The first sliding stand 13 is driven by the first screw rod 12 to make the blood-suctioning member 4 extend into or out of the blood sample testing tube. In this embodiment, the first sliding stand 13 is connected to one blood-suctioning member 4, and the blood-suctioning member 4 is used to suction the blood sample of different blood groups.

The reagent suction-driving mechanism 2 has a structure similar to the blood suction-driving mechanism 1, in this embodiment, specifically includes a second driving source 21, a second gear assembly, a second screw rod 22 and a second sliding stand 23, wherein the second driving source 21 is also fixed on the first mounting plate 6, specifically fixed to the lower end of the first mounting plate 6, arranged alongside the first driving source 11, the second driving source 21 can be a second motor, when implemented, and the driving shaft of the second motor 21 horizontally passes through the first mounting plate 6.

With the same structure as the first gear assembly, the second gear assembly includes a first gear 14 and a second gear 15, wherein the first gear 14 is fixed to the top end of the driving shaft of the second motor 21 (that is, the end that passes through the first mounting plate 6 ), driven to rotate axially by the driving shaft of the second motor 21, the second gear 15 encircles the second screw rod 22 and meshes perpendicularly with the first gear 14, that is, the second gear 15 rotates in a radial direction perpendicular to the axial direction while the first gear 14 rotates, that is, achieves a 90°turn. Of course, the invention is not limited to the gear assembly structure here, and other transmission structures that can realize the transmission between the driving source and the screw rod are applicable to the invention.

The second screw rod 22 is vertically arranged, that is, parallel or approximately parallel to the plane where the first mounting plate 6 is located, in this embodiment, it is threadedly connected to the second gear 15 and can be driven with the rotation of the second gear 15 to rotate with respect to the second gear 15 in a radial direction perpendicular to the rotation direction. Specifically, in this embodiment, the upper and lower ends of the second screw rod 22 are also rotatably connected to the second mounting plate 7, respectively. When implemented, the rotational connection between both the ends of the second screw rod 22 and the second mounting plate 7 can be achieved through the rotary bearing 8 in the same way.

The second sliding stand 23 is slidably connected to the second screw rod 22, and moves up and down along with the rotation of the second screw rod 22. Specifically, in this embodiment, the second sliding stand 23 is located between both the second mounting plates 7 and is arranged at the upper end of the second screw rod 22. Preferably, the second sliding stand 23 is also guided by a second sliding rail 62 to move in the vertical direction, and a second sliding groove matching with the second sliding rail 62 is arranged on the inner surface of the second sliding stand 23 abutting onto the second sliding rail 62 (not shown in the figures). In this embodiment, the second sliding rail 62 is vertically arranged on the first mounting plate 6.

The outer surface of the second sliding stand 23 (the side opposite to the second sliding groove) protrudes outwards to form a second fixing bump 231, and the reagent-suctioning member 5 vertically passes through the second fixing bump 231 and is fixed inside the second fixing bump 231. The second sliding stand 23 is driven by the second screw rod 22 to make the reagent-suctioning member 5 extend into or out of the reagent testing tube. In this embodiment, the second sliding stand 23 is connected to multiple reagent-suctioning members 5, preferably seven, and each reagent-suctioning member 5 suctions one reagent correspondingly. In implementation, multiple reagent-suctioning members 5 can all be connected to an integrated rod 9, and a connection opening 91 is provided on the integrated rod 9 at a position corresponding to each reagent-suctioning member 5.

The first motor 11, the second motor 21, the first mounting plate 6 and the second mounting plate 7 are all integrated and fixed on a base 10.

The rotationally-driving mechanism 3 is connected to the base 10, so as to realize the connection with both the blood suction-driving mechanism 1 and the reagent suction-driving mechanism 2, and is used to drive both the mechanisms 1, 2 to rotate in the horizontal direction, so that both the mechanisms 1, 2 move to the corresponding blood sample testing tube and reagent testing tube, respectively, which match with both the mechanisms 1, 2 to complete automatically driving the addition of the blood and reagents.

The rotationally-driving mechanism 3 specifically includes a third driving source 31 and a rotating shaft 32, when implemented, the third driving source 31 may be a third motor 31, which is connected to the rotating shaft 32 to drive the rotating shaft 32 to rotate. The rotating shaft 32 is connected to the base 10 described above, and the base 10 is driven to rotate in a horizontal direction, thereby synchronously driving the blood suction-driving mechanism 1 and the reagent suction-driving mechanism 2 to move in the horizontal direction.

Preferably, the first mounting plate 6 is also provided with a liquid level sensor 63 for detecting the liquid level inside the blood-suctioning member 4 and the reagent-suctioning member 5, so that the blood-suctioning member 4 and the reagent-suctioning member 5 suction blood samples and reagents to a preset liquid level, respectively.

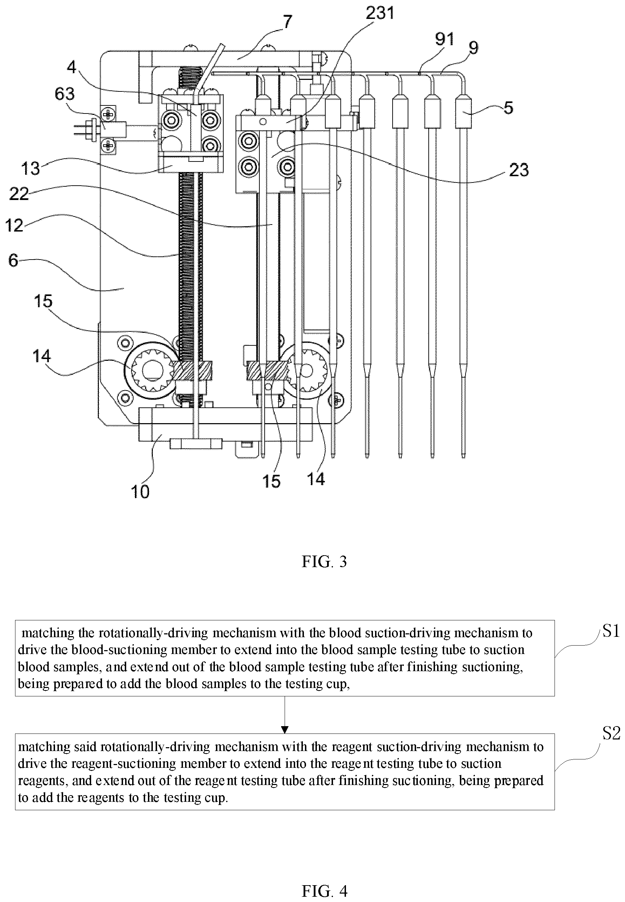

In combination with FIG. 4, a liquid-taking method disclosed in the embodiment of the invention specifically includes the following steps.

S1, Matching the rotationally-driving mechanism 3 with the blood suction-driving mechanism 1 to drive the blood-suctioning member 4 to extend into the blood sample testing tube to suction blood samples, and extend out of the blood sample testing tube after finishing suctioning, being prepared to add the blood samples to the testing cup.

Specifically, the third motor 31 drives the base 10 to make the blood-suctioning member 4 move above the blood sample testing tube to be suctioned, so that the lower end of the blood-suctioning member 4 is aligned with the blood sample testing tube. The first motor 11 drives the first screw rod 12 to rotate through the first gear assembly, and then drives the first sliding stand 13 through the first screw rod 12 to make the blood-suctioning member 4 move downwards and extend into the blood sample testing tube, so the blood-suctioning member 4 suctions a required amount of blood samples from the blood sample testing tube under the cooperative control of the liquid path system (not shown in the figures). After finishing suctioning, the first screw rod 12 drives the first sliding stand 13 to make the blood-suctioning member 4 move upwards to extend out of the blood sample testing tube. The third motor 31 drives the base 10 to make the blood-suctioning member 4 move above the testing cup, so that the lower end of the blood-suctioning member 4 is aligned with the testing cup. And then the first motor 11 drives the first screw rod 12 to rotate through the first gear assembly, and drives the first sliding stand 13 through the first screw rod 12 to make the blood-suctioning member 4 move downwards and extend into the testing cup, so the blood-suctioning member 4 releases the blood samples into the testing cup under the cooperative control of the liquid path system (not shown in the figures), and completes the process of automatically adding the blood samples into the testing cup. After completing the process, the blood-suctioning member 4 is reset by matching the rotationally-driving mechanism 3 with the blood suction-driving mechanism 1.

S2, Matching the rotationally-driving mechanism 3 with the reagent suction-driving mechanism 2 to drive the reagent-suctioning member 5 to extend into the reagent testing tube to suction reagents, and extend out of the reagent testing tube after finishing suctioning, being prepared to add the reagents to the testing cup.

Specifically, the third motor 31 drives the base 10 to make the reagent-suctioning member 5 move above the reagent testing tube to be suctioned, so that the lower end of the reagent-suctioning member 5 is aligned with the reagent testing tube. The second motor 21 drives the second screw rod 22 to rotate through the second gear assembly, and then drives the second sliding stand 23 through the second screw rod 22 to make the reagent-suctioning member 5 move downwards and extend into the reagent testing tube, so the reagent-suctioning member 5 suctions a required amount of reagents from the reagent testing tube under the cooperative control of the liquid path system (not shown in the figures). After finishing suctioning, the second screw rod 22 drives the second sliding stand 23 to make the reagent-suctioning member 5 move upwards to extend out of the reagent testing tube. The third motor 31 drives the base 10 to make the reagent-suctioning member 5 move above the testing cup, so that the lower end of the reagent-suctioning member 5 is aligned with the testing cup. And then the second motor 21 drives the second screw rod 22 to rotate through the second gear assembly, and drives the second sliding stand 23 through the second screw rod 12 to make the reagent-suctioning member 5 move downwards and extend into the testing cup, so the reagent-suctioning member 5 releases the reagents into the testing cup under the cooperative control of the liquid path system (not shown in the figures), and completes the process of automatically adding the reagents into the testing cup. After completing the process, the reagent-suctioning member 5 is reset by matching the rotationally-driving mechanism 3 with the reagent suction-driving mechanism 2.

## Claims

1. A liquid-taking device, **characterized in that** said device comprises a blood suction-driving mechanism (1), a reagent suction-driving mechanism (2), and a rotationally-driving mechanism (3), wherein said blood suction-driving mechanism (1) is connected to a blood-suctioning member (4), so as to drive said blood-suctioning member (4) to extend into or out of a blood sample testing tube, said reagent suction-driving mechanism (2) is connected to a reagent-suctioning member (5), so as to drive said reagent-suctioning member (5) to extend into or out of a reagent testing tube, said rotationally-driving mechanism (3) is connected to both said blood suction-driving mechanism (1) and said reagent suction-driving mechanism (2), used to drive both to rotate in the first direction,
wherein said blood suction-driving mechanism (1) includes a first driving source (11), a first gear assembly connected to said first driving source (11), a first screw rod (12) connected to said first gear assembly and a first sliding stand (13) connected to said first screw rod (12), said first sliding stand (13) is fixedly connected to said blood-suctioning member (4);
wherein said reagent suction-driving mechanism (2) includes a second driving source (21), a second gear assembly connected to said second driving source (21), a second screw rod (22) connected to said second gear assembly and a second sliding stand (23) connected to said second screw rod (22), said second sliding stand (23) is fixedly connected to said reagent-suctioning member (5).

2. The liquid-taking device according to claim 1, **characterized in that** both said blood suction-driving mechanism (1) and said reagent suction-driving mechanism (2) are fixed on a base (10), said base (10) is rotationally connected with said rotationally-driving mechanism (3).

3. The liquid-taking device according to claim 1, **characterized in that** said rotationally-driving mechanism (3) includes a third driving source (31) and a rotating shaft (32) connected with said third driving source (31), said rotating shaft (32) is fixedly connected to both said blood suction-driving mechanism (1) and said reagent suction-driving mechanism (2).

4. The liquid-taking device according to claim 1, **characterized in that** both said first gear assembly and said second gear assembly include a first gear (14) and a second gear (15) that mesh perpendicularly with each other, said first gear (14) is connected with the driving source (11, 21), and said second gear (15) is connected with the screw rod (12, 22).

5. The liquid-taking device according to claim 1, **characterized in that** said blood suction-driving mechanism (1) further includes a first sliding rail (61), and said first sliding stand (13) moves along said first sliding rail (61), driven by said first driving source (11), so as to drive said blood-suctioning member (4) to extend into or out of the blood sample testing tube.

6. The liquid-taking device according to claim 1, **characterized in that** the outer surface of said first sliding stand (13) protrudes outwards to form a first fixing bump (131), and said blood-suctioning member (4) vertically passes through said first fixing bump (131) and is fixed inside said first fixing bump (131).

7. The liquid-taking device according to claim 1, **characterized in that** said reagent suction-driving mechanism (2) further includes a second sliding rail (62), said second sliding stand (23) moves along said second sliding rail (62), driven by said second driving source (21), so as to drive said reagent-suctioning member (5) to extend into or out of the reagent testing tube .

8. The liquid-taking device according to claim 1, **characterized in that** the outer surface of said second sliding stand (23) protrudes outwards to form a second fixing bump (231), and said reagent-suctioning member (5) vertically passes through said second fixing bump (231) and is fixed inside said second fixing bump (231).

9. The liquid-taking device according to claim 8, **characterized in that** said reagent-suctioning member (5) is provided with an integrated rod (9) connected to said reagent-suctioning member (5) , and a connection opening (91) is provided on said integrated rod (9) at a position corresponding to each reagent-suctioning member (5).

10. A liquid-taking method which uses the liquid-taking device according to any one of claims 1-9, **characterized in that** said method comprises the following steps:
S1, matching said rotationally-driving mechanism (3) with the blood suction-driving mechanism (1) to drive the blood-suctioning member (4) to extend into the blood sample testing tube to suction blood samples, and extend out of the blood sample testing tube after finishing suctioning, being prepared to add the blood samples to the testing cup,
S2, matching said rotationally-driving mechanism (3) with the reagent suction-driving mechanism (2) to drive the reagent-suctioning member (5) to extend into the reagent testing tube to suction reagents, and extend out of the reagent testing tube after finishing suctioning, being prepared to add the reagents to the testing cup.

11. The liquid-taking method according to claim 10, **characterized in that** S1 specifically includes, said rotationally-driving mechanism (3) driving the blood suction-driving mechanism (1) to move to the blood sample testing tube, said blood suction-driving mechanism (1) driving the blood-suctioning member (4) to extend into the corresponding blood sample testing tube to suction a blood sample, after finishing suctioning, driving the blood-suctioning member (4) to extend out of the blood sample testing tube, and then said blood suction-driving mechanism (1) being driven by said rotationally-driving mechanism (3) to move to the testing cup, and being prepared to add the blood sample into the testing cup.

12. The liquid-taking method according to claim 10, **characterized in that** S2 specifically includes, said rotationally-driving mechanism (3) driving the reagent suction-driving mechanism (2) to move to the reagent testing tube, said reagent suction-driving mechanism (2) driving the reagent-suctioning member (5) to extend into the corresponding reagent testing tube to suction a reagent, after finishing suctioning, driving the reagent-suctioning member (5) to extend out of the reagent testing tube, and then said reagent suction-driving mechanism (2) being driven by said rotationally-driving mechanism (3) to move to the testing cup, and being prepared to add the reagent into the testing cup.

## Patentansprüche

1. Flüssigkeitsentnahmevorrichtung, **dadurch gekennzeichnet, dass** die Vorrichtung einen Blutansaug-Antriebsmechanismus (1), einen Reagenzienansaug-Antriebsmechanismus (2) und einen Drehantriebsmechanismus (3) umfasst, wobei der Blutansaug-Antriebsmechanismus (1) mit einem Blutansaugelement (4) verbunden ist, um das Blutansaugelement (4) so anzutreiben, dass es sich in ein Blutproben-Teströhrchen hinein oder aus diesem heraus erstreckt, der Reagenzienansaug-Antriebsmechanismus (2) mit einem Reagenzienansaugelement (5) verbunden ist, um das Reagenzienansaugelement (5) so anzutreiben, dass es sich in ein Reagenzien-Teströhrchen hinein oder aus diesem heraus erstreckt, der Drehantriebsmechanismus (3) mit sowohl dem Blutansaug-Antriebsmechanismus (1) als auch dem Reagenzienansaug-Antriebsmechanismus (2) verbunden ist und dazu verwendet wird, beide so anzutreiben, dass sie in der ersten Richtung drehen,
wobei der Blutansaug-Antriebsmechanismus (1) eine erste Antriebsquelle (11), eine erste Zahnradanordnung, die mit der ersten Antriebsquelle (11) verbunden ist, eine erste Gewindestange (12), die mit der ersten Zahnradanordnung verbunden ist, und einen ersten Gleitständer (13) beinhaltet, der mit der ersten Gewindestange (12) verbunden ist, wobei der erste Gleitständer (13) fest mit dem Blutansaugelement (4) verbunden ist;
wobei der Reagenzienansaug-Antriebsmechanismus (2) eine zweite Antriebsquelle (21), eine zweite Zahnradanordnung, die mit der zweiten Antriebsquelle (21) verbunden ist, eine zweite Gewindestange (22), die mit der zweiten Zahnradanordnung verbunden ist, und einen zweiten Gleitständer (23) beinhaltet, der mit der zweiten Gewindestange (22) verbunden ist, wobei der zweite Gleitständer (23) fest mit dem Reagenzienansaugelement (5) verbunden ist.

2. Flüssigkeitsentnahmevorrichtung nach Anspruch 1, **dadurch gekennzeichnet, dass** sowohl der Blutansaug-Antriebsmechanismus (1) als auch der Reagenzienansaug-Antriebsmechanismus (2) an einer Basis (10) befestigt sind, wobei die Basis (10) drehfest mit dem Drehantriebsmechanismus (3) verbunden ist.

3. Flüssigkeitsentnahmevorrichtung nach Anspruch 1, **dadurch gekennzeichnet, dass** der Drehantriebsmechanismus (3) eine dritte Antriebsquelle (31) und eine Drehwelle (32) beinhaltet, die mit der dritten Antriebsquelle (31) verbunden ist, wobei die Drehwelle ( 32) sowohl mit dem Blutansaug-Antriebsmechanismus (1) als auch dem Reagenzienansaug-Antriebsmechanismus (2) fest verbunden ist.

4. Flüssigkeitsentnahmevorrichtung nach Anspruch 1, **dadurch gekennzeichnet, dass** sowohl die erste Zahnradanordnung als auch die zweite Zahnradanordnung ein erstes Zahnrad (14) und ein zweites Zahnrad (15) beinhalten, die rechtwinklig ineinandergreifen, wobei das erste Zahnrad (14) mit der Antriebsquelle (11, 21) verbunden ist und das zweite Zahnrad (15) mit der Gewindestange (12, 22) verbunden ist.

5. Flüssigkeitsentnahmevorrichtung nach Anspruch 1, **dadurch gekennzeichnet, dass** der Blutansaug-Antriebsmechanismus (1) weiter eine erste Gleitschiene (61) beinhaltet und der erste Gleitständer (13) sich von der ersten Antriebsquelle (11) angetrieben entlang der ersten Gleitschiene (61) bewegt, um das Blutansaugelement (4) so anzutreiben, dass es sich in das Blutproben-Teströhrchen hinein oder aus diesem heraus erstreckt.

6. Flüssigkeitsentnahmevorrichtung nach Anspruch 1, **dadurch gekennzeichnet, dass** die Außenfläche des ersten Gleitständers (13) nach außen vorspringt, um einen ersten Befestigungsansatz (131) zu bilden, und das Blutansaugelement (4) vertikal durch den ersten Befestigungsansatz (131) hindurchgeht und innerhalb des ersten Befestigungsansatzes (131) befestigt ist.

7. Flüssigkeitsentnahmevorrichtung nach Anspruch 1, **dadurch gekennzeichnet, dass** der Reagenzienansaug-Antriebsmechanismus (2) weiter eine zweite Gleitschiene (62) beinhaltet, wobei sich der zweite Gleitständer (23) von der zweiten Antriebsquelle (21) angetrieben entlang der zweiten Gleitschiene (62) bewegt, um das Reagenzienansaugelement (5) so anzutreiben, dass es sich in das Reagenzien-Teströhrchen hinein oder aus diesem heraus erstreckt.

8. Flüssigkeitsentnahmevorrichtung nach Anspruch 1, **dadurch gekennzeichnet, dass** die Außenfläche des zweiten Gleitständers (23) nach außen vorspringt, um einen zweiten Befestigungsansatz (231) zu bilden, und das Reagenzienansaugelement (5) vertikal durch den zweiten Befestigungsansatz (231) hindurchgeht und innerhalb des zweiten Befestigungsansatzes (231) befestigt ist.

9. Flüssigkeitsentnahmevorrichtung nach Anspruch 8, **dadurch gekennzeichnet, dass** das Reagenzienansaugelement (5) mit einer integrierten Stange (9) bereitgestellt ist, die mit dem Reagenzienansaugelement (5) verbunden ist, und eine Verbindungsöffnung (91) an der integrierten Stange (9) an einer Position bereitgestellt ist, die jedem Reagenzienansaugelement (5) entspricht.

10. Flüssigkeitsentnahmeverfahren, das die Flüssigkeitsentnahmevorrichtung nach einem der Ansprüche 1-9 verwendet, **dadurch gekennzeichnet, dass** das Verfahren die folgenden Schritte umfasst:
S1, Abstimmen des Drehantriebsmechanismus (3) mit dem Blutansaug-Antriebsmechanismus (1), um das Blutansaugelement (4) so anzutreiben, dass es sich in das Blutproben-Teströhrchen hinein erstreckt, um Blutproben anzusaugen, und nach Abschluss des Saugens aus dem Blutproben-Teströhrchen heraus erstreckt, womit es darauf vorbereitet wird, die Blutproben in den Testbecher zu geben,
S2, Abstimmen des Drehantriebsmechanismus (3) mit dem Reagenzienansaug-Antriebsmechanismus (2), um das Reagenzienansaugelement (5) so anzutreiben, dass es sich in das Reagenzien-Teströhrchen hinein erstreckt, um Reagenzien anzusaugen, und nach Abschluss des Ansaugens aus dem Reagenzien-Teströhrchen heraus erstreckt, womit es darauf vorbereitet ist, die Reagenzien in den Testbecher zu geben.

11. Flüssigkeitsentnahmeverfahren nach Anspruch 10, **dadurch gekennzeichnet, dass** S1 insbesondere beinhaltet, dass der Drehantriebsmechanismus (3) den Blutansaug-Antriebsmechanismus (1) so antreibt, dass er sich zum Blutproben-Teströhrchen bewegt, der Blutansaug-Antriebsmechanismus (1) das Blutansaugelement (4) so antreibt, dass es sich in das entsprechende Blutproben-Teströhrchen hinein erstreckt, um eine Blutprobe anzusaugen, nach Abschluss des Ansaugens das Blutansaugelement (4) so antreibt, dass es sich aus dem Blutproben-Teströhrchen heraus erstreckt, und dann der Blutansaug-Antriebsmechanismus (1) vom Drehantriebsmechanismus (3) so angetrieben wird, dass er sich zum Testbecher bewegt und darauf vorbereitet ist, die Blutprobe in den Testbecher zu geben.

12. Flüssigkeitsentnahmeverfahren nach Anspruch 10, **dadurch gekennzeichnet, dass** S2 insbesondere beinhaltet, dass der Drehantriebsmechanismus (3) den Reagenzienansaug-Antriebsmechanismus (2) so antreibt, dass er sich zum Reagenzien-Teströhrchen bewegt, der Reagenzienansaug-Antriebsmechanismus (2) das Reagenzienansaugelement (5) so antreibt, dass es sich in das entsprechende Reagenzien-Teströhrchen hinein erstreckt, um ein Reagenz anzusaugen, nach Abschluss des Ansaugens das Reagenzienansaugelement (5) so antreibt, dass es sich aus dem Reagenzien-Teströhrchen heraus erstreckt, und dann der Reagenzienansaug-Antriebsmechanismus (2) vom Drehantriebsmechanismus (3) so angetrieben wird, dass er sich zum Testbecher bewegt und darauf vorbereitet ist, das Reagenz in den Testbecher zu geben.

## Revendications

1. Dispositif de prélèvement de liquide, **caractérisé en ce que** ledit dispositif comprend un mécanisme d'entraînement à aspiration de sang (1), un mécanisme d'entraînement à aspiration de réactif (2) et un mécanisme d'entraînement en rotation (3), dans lequel ledit mécanisme d'entraînement à aspiration de sang (1) est relié à un élément d'aspiration de sang (4), de manière à amener ledit élément d'aspiration de sang (4) à s'étendre dans ou hors d'un tube à essai d'échantillon de sang, ledit mécanisme d'entraînement à aspiration de réactif (2) est relié à un élément d'aspiration de réactif (5), de manière à amener ledit élément d'aspiration de réactif (5) à s'étendre dans ou hors d'un tube à essai de réactif, ledit mécanisme d'entraînement en rotation (3) est relié à la fois audit mécanisme d'entraînement à aspiration de sang (1) et audit mécanisme d'entraînement à aspiration de réactif (2), utilisé pour les entraîner tous deux en rotation dans la première direction,
dans lequel ledit mécanisme d'entraînement à aspiration de sang (1) inclut une première source d'entraînement (11), un premier ensemble engrenage relié à ladite première source d'entraînement (11), une première tige filetée (12) reliée audit premier ensemble engrenage et un premier support coulissant (13) relié à ladite première tige filetée (12), ledit premier support coulissant (13) est relié à demeure audit élément d'aspiration de sang (4) ;
dans lequel ledit mécanisme d'entraînement à aspiration de réactif (2) inclut une deuxième source d'entraînement (21), un second ensemble engrenage relié à ladite deuxième source d'entraînement (21), une seconde tige filetée (22) reliée audit second ensemble engrenage et un second support coulissant (23) relié à ladite seconde tige filetée (22), ledit second support coulissant (23) est relié à demeure audit élément d'aspiration de réactif (5).

2. Dispositif de prélèvement de liquide selon la revendication 1, **caractérisé en ce que** ledit mécanisme d'entraînement à aspiration de sang (1) et ledit mécanisme d'entraînement à aspiration de réactif (2) sont tous deux fixés sur une base (10), ladite base (10) est reliée de manière rotative audit mécanisme d'entraînement en rotation (3).

3. Dispositif de prélèvement de liquide selon la revendication 1, **caractérisé en ce que** ledit mécanisme d'entraînement en rotation (3) inclut une troisième source d'entraînement (31) et un arbre rotatif (32) relié à ladite troisième source d'entraînement (31), ledit arbre rotatif (32) est relié à demeure à la fois audit mécanisme d'entraînement à aspiration de sang (1) et audit mécanisme d'entraînement à aspiration de réactif (2).

4. Dispositif de prélèvement de liquide selon la revendication 1, **caractérisé en ce que** ledit premier ensemble engrenage et ledit second ensemble engrenage incluent tous deux un premier engrenage (14) et un second engrenage (15) qui s'engrènent perpendiculairement l'un avec l'autre, ledit premier engrenage (14) est relié à la source d'entraînement (11, 21), et ledit second engrenage (15) est relié à la tige filetée (12, 22).

5. Dispositif de prélèvement de liquide selon la revendication 1, **caractérisé en ce que** ledit mécanisme d'entraînement à aspiration de sang (1) inclut en outre un premier rail coulissant (61), et ledit premier support coulissant (13) se déplace le long dudit premier rail coulissant (61), entraîné par ladite première source d'entraînement (11), de manière à amener ledit élément d'aspiration de sang (4) à s'étendre dans ou hors du tube à essai d'échantillon de sang.

6. Dispositif de prélèvement de liquide selon la revendication 1, **caractérisé en ce que** la surface extérieure dudit premier support coulissant (13) fait saillie vers l'extérieur pour former une première bosse de fixation (131), et ledit élément d'aspiration de sang (4) passe verticalement à travers ladite première bosse de fixation (131) et est fixé à l'intérieur de ladite première bosse de fixation (131).

7. Dispositif de prélèvement de liquide selon la revendication 1, **caractérisé en ce que** ledit mécanisme d'entraînement à aspiration de réactif (2) inclut en outre un second rail coulissant (62), ledit second support coulissant (23) se déplace le long dudit second rail coulissant (62), entraîné par ladite deuxième source d'entraînement (21), de manière à amener ledit élément d'aspiration de réactif (5) à s'étendre dans ou hors du tube à essai de réactif.

8. Dispositif de prélèvement de liquide selon la revendication 1, **caractérisé en ce que** la surface extérieure dudit second support coulissant (23) fait saillie vers l'extérieur pour former une seconde bosse de fixation (231), et ledit élément d'aspiration de réactif (5) passe verticalement à travers ladite seconde bosse de fixation (231) et est fixé à l'intérieur de ladite seconde bosse de fixation (231).

9. Dispositif de prélèvement de liquide selon la revendication 8, **caractérisé en ce que** ledit élément d'aspiration de réactif (5) est doté d'une tige intégrée (9) reliée audit élément d'aspiration de réactif (5), et une ouverture de liaison (91) est prévue sur ladite tige intégrée (9) à une position correspondant à chaque élément d'aspiration de réactif (5).

10. Procédé de prélèvement de liquide qui utilise le dispositif de prélèvement de liquide selon l'une quelconque des revendications 1 à 9, **caractérisé en ce que** ledit procédé comprend les étapes suivantes :
S1, l'appariement dudit mécanisme d'entraînement en rotation (3) avec le mécanisme d'entraînement à aspiration de sang (1) pour amener l'élément d'aspiration de sang (4) à s'étendre dans le tube à essai d'échantillon de sang pour aspirer des échantillons de sang, et à s'étendre hors du tube à essai d'échantillon de sang après avoir terminé l'aspiration, prêt à ajouter les échantillons de sang dans la coupelle d'essai,
S2, l'appariement dudit mécanisme d'entraînement en rotation (3) avec le mécanisme d'entraînement à aspiration de réactif (2) pour amener l'élément d'aspiration de réactif (5) à s'étendre dans le tube à essai de réactif pour aspirer des réactifs, et à s'étendre hors du tube à essai de réactif après avoir terminé l'aspiration, prêt à ajouter les réactifs dans la coupelle d'essai.

11. Procédé de prélèvement de liquide selon la revendication 10, **caractérisé en ce que** S1 inclut plus particulièrement ledit mécanisme d'entraînement en rotation (3) entraînant le déplacement du mécanisme d'entraînement à aspiration de sang (1) vers le tube à essai d'échantillon de sang, ledit mécanisme d'entraînement à aspiration de sang (1) amenant l'élément d'aspiration de sang (4) à s'étendre dans le tube à essai d'échantillon de sang correspondant pour aspirer un échantillon de sang, après avoir terminé l'aspiration, amenant l'élément d'aspiration de sang (4) à s'étendre hors du tube à essai d'échantillon de sang, puis ledit mécanisme d'entraînement à aspiration de sang (1) étant amené par ledit mécanisme d'entraînement en rotation (3) à se déplacer vers la coupelle d'essai, et prêt à ajouter l'échantillon de sang dans la coupelle d'essai.

12. Procédé de prélèvement de liquide selon la revendication 10, **caractérisé en ce que** S2 inclut plus particulièrement ledit mécanisme d'entraînement en rotation (3) entraînant le déplacement du mécanisme d'entraînement à aspiration de réactif (2) vers le tube à essai de réactif, ledit mécanisme d'entraînement à aspiration de réactif (2) amenant l'élément d'aspiration de réactif (5) à s'étendre dans le tube à essai de réactif correspondant pour aspirer un réactif, après avoir terminé l'aspiration, amenant l'élément d'aspiration de réactif (5) à s'étendre hors du tube à essai de réactif, puis ledit mécanisme d'entraînement à aspiration de réactif (2) étant amené par ledit mécanisme d'entraînement en rotation (3) à se déplacer vers la coupelle d'essai, prêt à ajouter le réactif dans la coupelle d'essai.
